# EUROPEAN PATENT APPLICATION

(11) **EP 3 034 615 A1**
(43) Date of publication of application: **22.06.2016**
(21) Application number: 14307038.1
(22) Date of filing: 15.12.2014
(51) Int. Cl.: C12Q 1/02, G01N 33/72

(54) **Sensitive bacterial-growth-based protoporphyrin detection test**

(71) Applicant: INSTITUT NATIONAL DE LA RECHERCHE AGRONOMIQUE (INRA), 75007 Paris (FR)
(72) Inventor: Gruss, Alexandra, 91400 Orsay (FR); Halpern, David, 91160 Longjumeau (FR)
(74) Representative: Coralis Harle

(57) **Abstract**

A method for detecting the presence of protoporphyrin in a sample, said method comprising steps of:
a) cultivating heme auxotroph bacteria (HAB) in a protoporphyrin-lacking culture medium, in the presence of said sample, for a determined time period,
b) estimating the extent of HAB growth issued at step a), to obtain a sample signal, and
c) identifying the presence of protoporphyrin in said sample, by comparing the sample signal obtained at step b) with at least one reference signal.

## Description

### FIELD OF THE INVENTION

The present invention relates generally to a method and to a device for detecting the presence of protoporphyrin (heme and/or protoporphyrin IX, PPIX) in a sample. More particularly, the present invention relates to a highly sensitive and specific method and device for detecting heme in a complex sample, based on a bacteriological growth assay. The principles of the present invention may be used for detecting occult blood, hemoglobin, heme or in some embodiments, if present, PPIX, in various complex samples.

### BACKGROUND OF THE INVENTION

Heme detection in complex biological samples, in particular in feces or other intestinal samples including gastro-intestinal fluids, and in urine, is a primary tool in medicine where it is a widely used indicator of occult (hidden) blood and underlying diseases.

Indeed, occult blood in feces, often present in minute amounts undetectable to the naked eye, is an early sign of intestinal inflammation, colorectal cancer, adenomas and polyps. As colorectal cancers are ranked second among cancer-related deaths in both women and men, reliable detection methods are of particular importance. Moreover, unlike other forms of cancer, its early diagnosis and treatment favors a high cure rate of over 90% of patients; if the disease is not detected until later stages, the cure rate drops drastically to 25% or less (Marbet U, 2006, Forum Med Suisse, 6:291-8).

Colonoscopy is an effective method for detecting colorectal cancer, but this procedure is costly and is not convenient as an initial screening test; furthermore, it is invasive and acceptance by patients is poor. A recently developed method is based on a DNA screening approach; however, the method is costly, and involves collection of about 300 grams of fecal samples, which may receive limited patient cooperation. Fecal occult blood testing is therefore the screening method of choice because it allows large-scale detection of risk of colorectal cancer at an early stage.

Several tests and procedures for detecting occult blood in fecal matter have been developed. The most widely used test, which relies on a biochemical assay, is offered by Beckman Coulter, Inc. (Fullerton, California) under the trademark Hemoccult® (disclosed in U.S. Patent No. 3,996,006). Hemoccult-type tests are used worldwide (cohorts in efficacy tests usually involve 10,000 or more individuals per group) and have been recommended to essentially the population over the age of 50 as an indicator of colorectal cancer, which can cause occult bleeding. Briefly, a thin smear of fecal material is applied to one side of guaiac paper by the patient and sent to a medical laboratory for analysis. A developing solution, such as hydrogen peroxide, is then applied to the opposite side of the guaiac paper. If blood is present in the fecal material, the guaiac reaction will color the paper blue. Testing a fecal specimen for occult blood is based on the principle that heme originating from blood hemoglobin will function as a catalyst and cause oxygen to be liberated from an oxygen donor, with the liberated oxygen thus causing a color change in the chromogenic substance (guaiac). For example, the most commonly used heme detection test is Hemoccult II® (Beckman Coulter Inc.), which reportedly detects around 300 µg hemoglobin per gram feces, which is equivalent to about 12 µg heme/gram feces. This appears to be the lowest possible signal giving a positive response.

Guaiac-based "Hemoccult" tests are rapid, simple and disposable. However sensitivity and specificity are main limitations of these tests, which may lead to a relatively high number of false negative or positive test results. These pitfalls may be inherent to the use of biochemical tests, where activities can be affected by other feces components, including bile. Procedures were developed to overcome these problems, but current heme detection methods are time-consuming, and detection thresholds may be limited. Another problem associated with these guaiac tests is instability of the test results. Indeed, test paper must be examined within about one minute after addition of the developer to prevent inaccurate interpretation of test results due to color fading. Lastly, this test requires the patient to limit consumption of commonly consumed foods and drugs, including red meat, vitamin C, and aspirin several days prior to testing (see product instructions for Hemoccult II® as published in 2009 by Beckman Coulter Inc. and available at https://www.beckmancoulter.com/wsrportal/wsr/diagnostics/clinical-products/rapid-diagnostics/hemoccult-sensa/index.htm#2/10//0/25/1/0/asc/2/64115//C_TECHNICALDOCS/0/0//1/).

A similar screening test, Gastroccult® (Beckman Coulter Inc.), which is used to monitor heme in gastric fluids, further requires avoidance of low pH, high drug concentrations, metal ions, or plant peroxidases as may be found in food, and could lead to false positive results (see product instructions for Gastroccult® by Beckman Coulter Inc. as published in 2011 and available at http://www.cliawaived.com/web/items/pdf/BKM-SK-66040_Gastroccult_Test_Kit_insert∼1251file1.pdf).

Immunochemical tests to detect occult blood have also been reported. These more recent tests (for example OC-SENSOR®, Eiken) rely on immunological detection of human hemoglobin. Their sensitivity appears to be higher than biochemical tests (with a reported range of 10-200 µg hemoglobin per gram feces). However, while these tests are specific for human hemoglobin, a possible drawback is hemoglobin degradation, which might lead to false negatives. Finally, interference due to fecal matter in signal detection was previously reported (Väänänen and Tenhunen, Clin Chem., 1988, 34:1763-6), but to our knowledge has not been considered in reports on OC-SENSOR® efficacy. Lastly, immunological tests are more expensive than the more commonly used guaiac-based tests.

Hematuria (presence of red blood cells in urine) may be a primary symptom of underlying disease, and heme detection is routinely used as a primary diagnostic (as mentioned in the article "Hematuria" from Sanjeev Gulati, Sanjay Gandhi and Deogracias Pena, published in May 2014 on the Medscape website, at the following internet address http://emedicine.medscape.com/article/981898-overview?src=wgt_edit_news_lsm&lc=int_mb_1001). While the currently used test referred to as Multistix® 8SG (Siemens Healthcare Diagnostic Inc. Tarrytown, NY) is sensitive, the exceedingly low amounts of heme that are clinically relevant still leave the possibility that red blood cells are present below the level of detection.

PPIX, like heme, has been the target of development as an indicator of underlying diseases, including cancers, but the cost of detection is high (Silva et al., 2013, J Fluorescence 23(1):131-5).

We considered the need for a reliable, easy to use method of detecting heme, even in complex samples, that would combine a high sensitivity with a high specificity. Preferentially the method should allow either automated or human reading, depending on implementation conditions.

### SUMMARY OF THE INVENTION

The present invention provides a technical solution to the above-mentioned problems and relates to a method for detecting the presence of protoporphyrin, in a sample.

In the present application, the following definitions will be used:

By "heme", it is intended the association of a Fe²⁺ ferrous ion in the center of the protoporphyrin IX ring (Fe-PPIX). Heme is formed by enzymatic insertion of the Fe²⁺ ion in the PPIX ring. Hemin, in which Fe³⁺ ions are present, rather than Fe²⁺ ions, is also referred to here as heme for simplicity.

By "protoporphyrin IX" (PPIX), it is thus intended the immediate biological precursor of heme, consisting of the porphyrin ring without the iron ion.

In this invention, heme and/or PPIX, which differ by an iron ion, are collectively referred to as protoporphyrins.

The method for detecting the presence of protoporphyrin (heme and/or PPIX) according to the invention is based on the fact that heme-requiring bacteria (referred to as heme auxotroph bacteria or HAB) are unable to grow unless heme is present in the growth medium. Importantly, heme (or PPIX for some HAB subtypes) is the growth-limiting factor. Other growth-stimulating factors of HAB will have no visible effect unless heme, or in some case heme or PPIX is present. As such, the extent of bacterial growth in the present detection system depends upon the amount of heme and/or PPIX present in the sample.

The said method comprises the following steps:
a) cultivating HAB in a protoporphyrin-lacking culture medium, in the presence of a sample to be analyzed, for a determined time period,
b) estimating the extent of HAB growth issued after step a), referred to here as the sample signal, and
c) identifying the presence of protoporphyrin in said sample, typically by comparing the sample signal obtained at step b) with at least one reference signal.

To the authors' knowledge this method is, for the first time, based on bacterial growth, rather than on a biochemical or immunochemical assay. Indeed the authors have identified, as demonstrated in the examples detailed in the present application, that bacteria that need exogenous heme and/or PPIX for growth can be used in a method to detect said molecules in a sample, with high specificity and excellent sensitivity.

In all embodiments of the invention, the amount of protoporphyrin in a sample is correlated to the extent of HAB growth ("sample signal").

In one embodiment, the protoporphyrin to be detected in the sample is heme. Consequently, heme-specific HAB are used and cultured at step a) in a heme-lacking medium. In this embodiment, growth of the heme-specific HAB is stimulated by heme but not by PPIX. Heme-specific HAB are preferentially chosen among native or recombinant strains that are deficient in enzymes that catalyze iron insertion into protoporphyrin IX.

In some embodiments, at step c) of the method, at least one negative reference signal and/or at least one positive reference signal are used.

The invention can also comprise a further step of semi-quantitative estimation of the amount of heme in the sample, by comparing the sample signal obtained at step b) with at least one benchmark reference signal. Said at least one benchmark reference signal is obtained by cultivating HAB in the protoporphyrin-lacking culture medium, for a determined time period, in the presence of a predetermined protoporphyrin concentration.

According to the invention, HAB can be chosen among the following genera or their recombinant HAB derivatives: *Bacteroides, Haemophilus, Lactobacillus, Lactococcus, Enterococcus,* or heme auxotrophic mutants of heme prototrophs *Escherichia* and *Bacillus.*

The present invention also provides a method for detecting the presence of blood in a sample, said method comprising:
- steps of the method as specified here-above, and
- a further step of identifying the presence of blood in said sample, in regard to step c) of identifying protoporphyrin in the sample.

In some embodiments of the invention the sample is typically a complex sample, such as preferentially a food product (for example milk), fecal material, a gastro-intestinal fluid, or urine.

Typically, the sample is heat-inactivated prior to step a) of the detection method of the invention.

The present invention also relates to a device for detecting the presence of protoporphyrin in a sample, and suitable for implementing the method as previously mentioned.

In one embodiment, the device according to the invention contains HAB suspended in a protoporphyrin-lacking culture medium and at least one well intended to receive a sample, wherein said HAB can access to said sample. Typically, the protoporphyrin-lacking culture medium is in the form of a gel.

In some embodiments, the device can further contain at least one other well, which is intended to receive a reference sample such as a benchmark reference sample.

The present invention also describes a kit comprising:
- a device according to the invention,
- at least one benchmark reference sample having a predetermined protoporphyrin concentration, and
- a strain of HAB to be mixed with the protoporphyrin-lacking medium.

The present invention also relates to the use of a device or a kit according to the invention for preliminary screening for potential inflammation or disease.

The present invention also relates to the use of a device or a kit as previously described for detection of mastitis in farm animals, especially those used for milk production.

In some other embodiments, the invention also relates to the use of a device or a kit as previously described for detecting the presence of meat-derived ingredients in food products.

### DESCRIPTION OF THE DRAWINGS

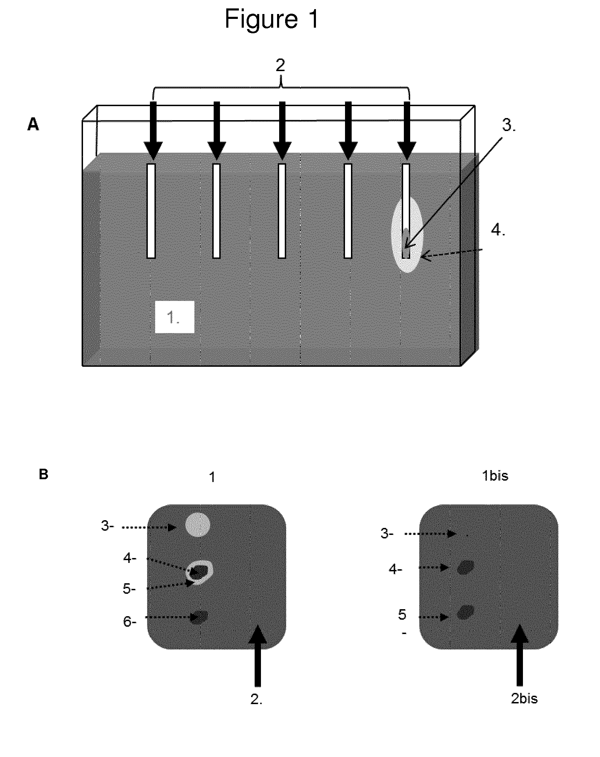

Figure 1 -Schematic depiction of heme or heme/PPIX detection devices.
1A: Prototype **Gel** device setup for protoporphyrin detection by HAB growth stimulation (1-Indicator HAB embedded in gel. The gel, which contains nutrients for bacterial growth, but without protoporphyrin, is mixed with a HAB strain according to the invention (such as the *Bacteroides* strain *B. thetaiotaomicron:* referred to as Bt), 2- wells to deposit samples, 3-sample, 4- "halo" corresponding to stimulated HAB growth. 1 B: Prototype **Plate** device setup for protoporphyrin detection by HAB growth stimulation. Left: 1- Petri plate containing appropriate HAB solid medium but without protoporphyrin. 2- A HAB overlay, containing a HAB strain according to the invention (such as *Bacteroides* strains *B. thetaiotaomicron,* or *Lactococcus lactis)* and growth medium devoid of protoporphyrin, is poured over "1" and then allowed to solidify 3- heme-positive control sample, 4- Feces sample deposited upon the overlay, 5- "halo" developing during growth corresponding to stimulated HAB growth, 6- no stimulation is obtained if the sample is devoid of protoporphyrin. Right: 1 bis: Petri plate containing a negative control reference strain whose growth in not stimulated by heme. 2bis Overlay as in "2" except that the HAB strain does not respond to heme.

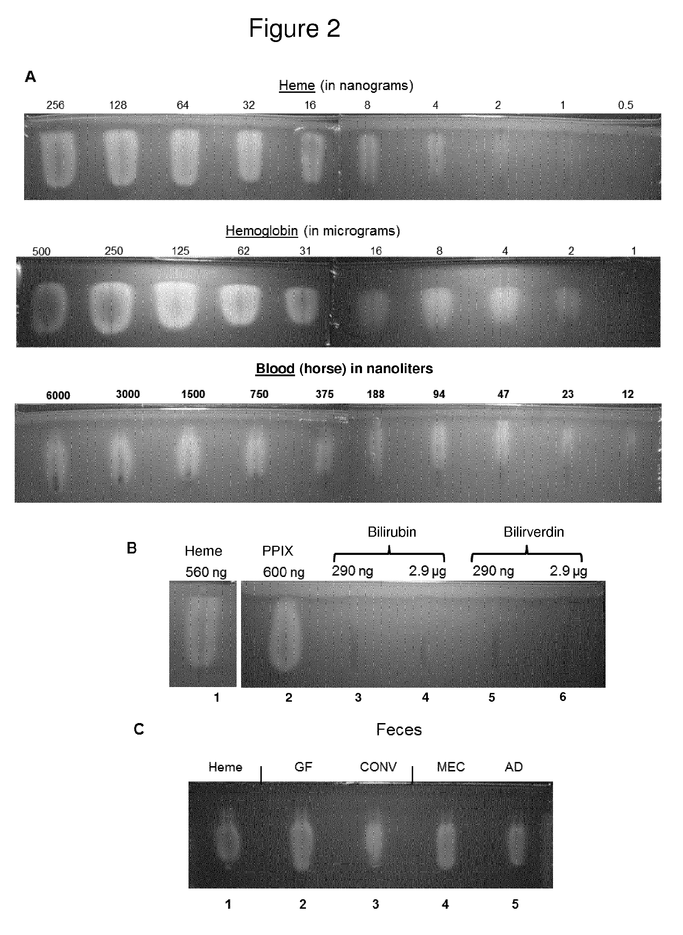

Figure 2: Threshold of Bt responses to (A) purified heme (upper panel), hemoglobin (middle panel), or blood (lower panel), (B) heme breakdown products, or (C) heme within a complex feces sample. - 2A The amount of purified heme needed to stimulate Bt growth was determined by loading 2-fold dilutions of pure heme ranging from 256 to 0.5 nanograms (ng) per well (from left to right: 256; 128; 64; 32; 16; 8; 4; 2; 1; and 0.5).; pure hemoglobin ranging from 500 to 1 microgram (µg) per well (from left to right: 500; 250; 125; 62; 31; 16; 8; 4; 2; 1), or blood, ranging from 6000 to 12 nanoliters (nl) per well (from left to right: 6000; 3000; 1500; 750; 375; 188; 94; 47; 23; and 12). The lowest amount of heme to clearly stimulate Bt growth was around 1 ng. The lowest amount of hemoglobin to clearly stimulate Bt growth was around 2 µg. The lowest amount of blood to clearly stimulate Bt growth was around 12 nl. 2B- Heme (560 ng) (1), PPIX (600 ng) (2), bilirubin (3-4) (290 to 2900 ng/sample), and biliverdin (5-6) (290 to 2900 ng/sample) were deposited in the test wells. Heme and PPIX, but not the heme breakdown products, were detected. - 2C: Feces from mice and humans stimulate Bt growth. The stimulatory effects are observed with feces from germfree animals and newborns, and also with feces from conventionally colonized mice and adult humans. The gel contained Bt in M17-0.5 % glucose (M17-glu) medium. The deposited sample (about 5 mg sample of feces per well) are as follows from left to right (wells 2-5): feces from healthy germfree and conventional mice (GF and CONV respectively), and from human meconium and adult (M and AD respectively). 120 ng of heme was loaded in well 1 and used as reference sample.

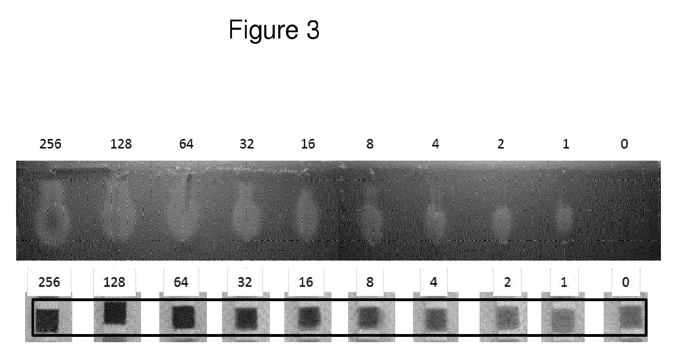

Figure 3. Detection threshold of heme in urine samples using the "HemeScreen" test of the invention and the commercially available Multistix® 8SG test. The urine sample was spiked with specified amounts of heme (from 0 to 256 ng) as indicated in the figure.
3 UPPER: "HemeScreen" test of the invention, the amount of heme present in urine needed to stimulate growth of Bt was done as described in Figure 2A. The lowest heme concentration detected as stimulating Bt growth was about 1 ng.
3 LOWER: Multistix® 8SG test: urine containing heme at the same concentrations as above tested with the Multistix® 8SG dipstick routinely used to test hematuria and processed as recommended by manufacturer. The lowest heme concentration detected by Multistix® 8SG was 8 ng. "HemeScreen" therefore shows about 8-fold greater sensitivity compared to Multistix® 8SG.

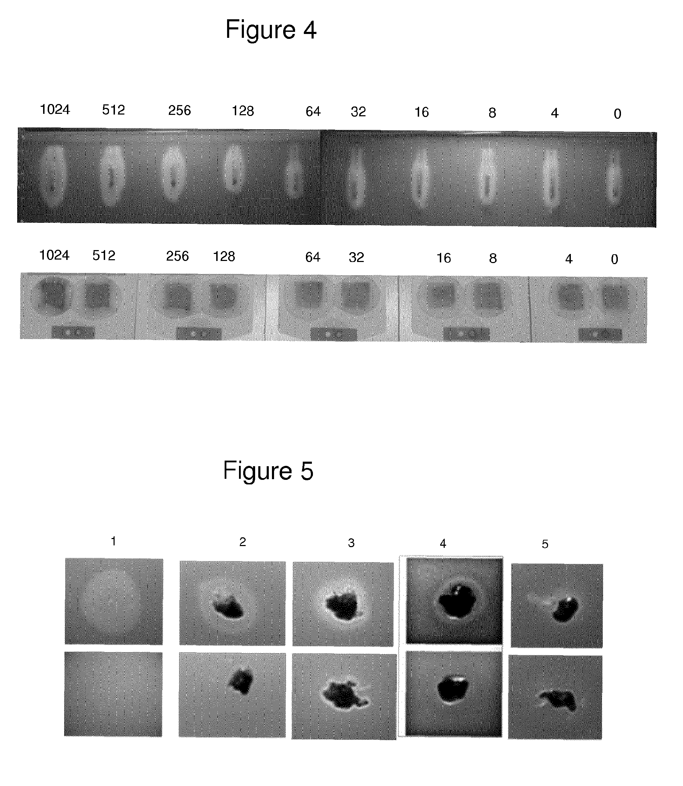

Figure 4. Using FECES as the complex sample. The samples were tested for heme using the "HemeScreen" test according to the invention and the Hemoccult II® test.
4 UPPER: "HemeScreen" test: Dilutions of heme were prepared in feces, such that each heme concentration (from 0 to 1024 ng) was prepared in 5 mg feces that were suspended in 50 microliters (µl) of 0.9 % NaCl. Heme was detected in all samples, including the sample to which heme was added, indicating that the feces sample already contains low levels of heme. A progressive increase in heme levels is detected when 128 ng or more heme are added to samples. This would indicate that amounts present in the healthy individual are less than 128 ng heme per 5 mg feces sample, which would allow detection of an incremental increase.
4 LOWER: Hemoccult II® test: The same samples were deposited on the Hemoccult II® test and processed as recommended by manufacturer. The lowest heme-containing feces suspension to be detected by Hemoccult II® was in the range of 512-1024 ng heme. Unlike the "HemeScreen" test of the invention, Hemoccult II® does not detect heme starting in the range present in normal individuals. Moreover, feces quenches the positive signal when Hemoccult II® is used (compare Figure 2 and 3B, threshold LOWER panels), whereas quenching does not occur using "HemeScreen" (compare Figure 2 and 4, threshold UPPER panels).
Note that while heme detected by Hemoccult II® is at around 1000 ng/ per sample, lower amounts of heme constitute a potential risk (see http://www.bestcarelab.com/testsmenu/78.htm, and Wong et al., 2012, Int. J. Colorectal Dis. 27:1657-64). The "HemeScreen" test thus detects heme at the lower risk level.

Figure 5: Heme detection using the plate set-up described in Figure 1B. Heme detection in complex samples with the "HemeScreen" plate set-up of the invention. The test uses *L. lactis* WT as indicator (upper panel), and *L. lactis cydA*-minus as negative control indicator (lower panel). Plates were prepared using M17-glu 0.5 %. They were overlaid with 10 ml soft agar (0.75 % agar and M17-glu 0.5 %) containing ∼10⁷ CFU/ml of *L. lactis,* and allowed to solidify. Reference heme sample (120 ng) (1) and test samples were then deposited on top of the plates, and plates were then incubated aerobically overnight at 30°C. Plates were then photographed. Shown here are the areas containing heme-positive reference samples (1), mouse feces (germfree (2); conventional: (3) and human feces, newborn: (4), adult: (5)).

### DETAILED DESCRIPTION OF THE INVENTION

The present invention provides a bacteriological-based technology, in particular a method (or a test) and a device, for detecting the presence of protoporphyrin in a sample.

The properties of heme iron as a transition metal that cycles between redox states make it the central factor in various biological processes. Heme is most commonly recognized as a component of hemoglobin, the red pigment in blood, but is also found in a number of other biologically important hemoproteins, such as myoglobin, cytochrome, catalase, and endothelial nitric oxide synthase. In the hemoglobin protein, heme is responsible for its oxygen-carrying properties and therefore constitutes the bioactive component of blood. In mammals, hemoglobin makes up about 96% (by weight) of the dry content of red blood cells.

The present invention is intended for detecting the presence of protoporphyrin (*i.e.,* heme and PPIX). Heme can be in its isolated form (free heme) or in complexed form, for example bound to a protein, such as in hemoglobin and other hemoproteins as mentioned above. Therefore hemoglobin can also be detected according to the method of the invention.

In a particular embodiment, the present invention is therefore intended to detect specifically the presence of heme, which can also be isolated (free heme), or in complexed form.

By "detecting the presence of protoporphyrin", it is intended notably a qualitative test for protoporphyrin detection in a sample, *i.e.* to determine the presence or the absence of protoporphyrin in said sample.

Similarly, by "detecting the presence of heme", it is also intended a semi-quantitative means of heme detection in a sample, *i.e.,* the detection of the amount of protoporphyrin in a sample. Indeed, the performances of the method, or of the device according to the invention, also allow embodiments for achieving semi-quantitative heme measurement in a sample.

Detection of protoporphyrin, and notably detection of heme, can be advantageously directly correlated to detection of blood, such that the method and the device of the invention can be used for detecting blood in a sample.

As mentioned above, the invention is based on the use of HAB, cultured in a protoporphyrin-lacking medium.

The sample to be analyzed is deposited into such HAB cultured in such protoporphyrin-lacking medium.

If the said sample contains protoporphyrin, HAB growth is stimulated. The authors have found that the extent of bacterial growth can directly, and in a sensitive way, be correlated to the presence of protoporphyrin in the sample. Indeed, the authors determined that the invention allows detection of 100 ng of heme per gram of sample. This invention is therefore about 60 times more sensitive than classical biochemical Hemoccult® tests for testing feces samples.

The present invention is highly specific for the detection of heme and PPIX. In particular, heme breakdown products, such as bilirubin or biliverdin that are commonly present in feces are not detected by the method of the invention, therefore avoiding false positive results. Furthermore, contrary to immunochemical tests, the method of the invention is insensitive to hemoglobin degradation, thereby decreasing false negative results.

It is noted that several samples can be tested in parallel. As such, gels containing HAB in a protoporphyrin-lacking culture medium can be prepared in parallel to detect the presence of protoporphyrin in independent samples (such as feces samples), together with standardized heme controls (reference samples).

### Heme auxotroph bacteria (HAB):

HAB are bacteria that lack some or all of the enzymes necessary to synthesize their own heme.

Among them, three categories of HAB are distinguished, having:
(i) a quasi-absolute heme requirement and therefore depending on exogenous heme (or PPIX) acquisition, *i.e.* requiring heme (or PPIX) for bacterial growth,
(ii) an optional heme requirement, *i.e.* restricted bacterial growth in the absence of exogenous heme (or PPIX), and
(iii) no heme requirement.

According to the invention, HAB implemented in the method of the invention are chosen among categories (i) and (ii) here-above, *i.e.* (i) a quasi-absolute heme requirement or (ii) an optional heme requirement. According to the invention, by "heme requirement", it is intended heme requirement to obtain growth or at least significant bacterial growth, as already known by the literature (see notably Varel VH & Bryant MP, 1974, Appl Microbiol., 28:251-7).

Some HAB encode enzymes that catalyze iron insertion into PPIX to form heme. In such HAB, exogenous PPIX may, like heme, stimulate HAB growth (see for example Sperry JF et al., 1977, Appl Environ Microbiol., 34:386-90). Therefore, such HAB strains can be used in the method of the invention to detect, without differentiation, the presence of heme or PPIX in a sample. It is noted that PPIX detection can be of particular interest for the detection of particular diseases, such as blood loss in the upper gastrointestinal (Gl) tract, as well as various porphyria.

Alternatively, using a HAB strain that has no ability to catalyze iron insertion into PPIX to form heme, renders the test totally heme-specific. Therefore, in a particular embodiment of the present invention, heme-specific HAB strains lacking (either naturally or through genetic manipulation) the ability to catalyze iron insertion into PPIX, can be used for the specific detection of heme in a sample. More preferentially such HAB strains lack the enzymatic activity needed for PPIX utilization by HAB. Typically such enzymatic activity converts protoporphyrin IX into heme by catalyzing the reaction: [protoporphyrin + (Fe²⁺) ↔ protoheme + 2 H⁺]. Recent studies in *Escherichia. coli (E. coli*) (Letoffe S et al., 2009, PNAS, 106:11719-24) revealed a novel enzyme that is needed to remove iron from heme, for which a homolog having potential reversible activity is also present in *Bacteroides.* Therefore inactivation of this putative enzyme, if present in the selected HAB, could be performed to restrict detection of protoporphyrin to heme.

HAB (including heme-specific HAB) are found in different ecosystems, and comprise Gram-positive or Gram-negative bacteria. HAB (including heme-specific HAB) may be aerobic, anaerobic or anaerobic aero-tolerant organisms (see Gruss A. et al., 2012, Adv Microb Physiol., 61:69-124).

By "genetic manipulation", it is intended notably genetic manipulation of heme producing bacteria *(e.g.: Escherichia coli* or *Bacillus subtilis),* to generate recombinant bacteria having a quasi-absolute heme requirement or an optional heme requirement, or of HAB (e.g. *Bacteroides fragilis* or B. *thetaiotaomicron)* to generate derivatives having deficiency in the ability to catalyze iron insertion into PPIX (for example ferrochelatase deficiency) and that are stimulated only by heme, and not by PPIX. Such genetic manipulations, to obtain heme-requiring or fully heme-specific isolates, are well known in the art. Heme auxotrophy and heme specificity, according to the invention, can therefore be native or due to genetic manipulation.

In some embodiments of the method, HAB (including heme-specific HAB) are luminescent or express a fluorescent protein. Preferentially, said HAB have been genetically modified to express a fluorescent protein. Such HAB could be used to facilitate signal detection directly by eye visualization, or by using typically a fluorimeter.

According to the invention, appropriate HAB (including heme-specific HAB) can be selected among HAB (including heme-specific HAB, native or resulting from genetic manipulation) of the following genera: *Streptococcus, Bacteroides, Haemophilus, Lactobacillus, Lactococcus, Escherichia, Bacillus, or their recombinant derivatives.* More preferentially HAB are chosen from the group of HAB from a genus selected from *Bacteroides, Lactobacillus, Lactococcus,* or their recombinant derivatives, or recombinant *Bacillus or Escherichia coli.*

For example, HAB (including heme-specific HAB, native or resulting from genetic manipulation) can be selected from among the following genera or species: *Streptococcus,* for example *Streptococcus agalactiae, Streptococcus dysgalactiae, Streptococcus suis,* and *Streptococcus uberis, Leuconostoc spp., Enterococcus faecalis; Lactobacillus,* for example, *Lactobacillus rhamnosus, Lactobacillus reuteri, Lactobacillus johnsonii, Lactobacillus plantarum, Lactobacillus casei, Lactobacillus brevis, Lactobacillus sakei, Lactobacillus salivarius,* and *Lactobacillus gasseri; Bacteroides,* for example *Bacteroides fragilis and Bacteroides thetaiotaomicron.*

Preferentially, HAB (including heme-specific HAB, native or resulting from genetic manipulation) can be selected from the group consisting of *Bacteroides thetaiotaomicron, Bacteroides fragilis, Haemophilius influenzae, Lactococcus lactis,* recombinant *Bacillus subtilis* or recombinant *Escherichia coli.* In particular, it can be selected from the group consisting of native or recombinant *Bacteroides thetaiotaomicron, Bacteroides fragilis,* or *Lactococcus lactis.*

In a preferred embodiment of the method, anaerobic HAB (including heme-specific HAB) are chosen from the genus *Bacteroides;* in particular *Bacteroides fragilis* and/or *Bacteroides thetaiotaomicron* are used according to the invention. These bacteria are particularly interesting, because they grow rapidly and because low concentrations of heme are sufficient to stimulate their growth.

### Method of the invention

The method according to the invention, for detecting the presence of protoporphyrin in a sample comprises steps of:
a) cultivating heme auxotroph bacteria (HAB) in a protoporphyrin-lacking culture medium, in the presence of said sample for a determined lapse of time,
b) estimating the extent of HAB growth issued at step a), to obtain a sample signal, and
c) comparing said sample signal obtained at step b), with at least one reference signal, to identify the presence, and in some cases, the amount of protoporphyrin in said sample.

### Step a) of the method

By "cultivating", it is intended conditions and culture parameters suitable for HAB growth, in case protoporphyrin is supplied *via* the sample to culture medium. The sample is deposited within a defined and limited space, which may be a well, or a spot deposited on solid medium. Typically HAB are re-suspended in a protoporphyrin-lacking culture medium, before being cultivated according to step a).

"By protoporphyrin-lacking medium" it is intended that the medium does not contain protoporphyrin (heme and/or PPIX), which is to be detected in the sample. When "heme-specific" HAB as described above are used, the culture medium should be devoid of heme. Alternatively, when HAB encode enzymes that catalyze iron insertion into PPIX to form heme, the culture medium should be devoid of both heme and PPIX.

The incubation period for the HAB culture should be theoretically sufficient for detecting stimulation of bacterial growth, if any.

Said incubation period depends on several parameters such as HAB type, HAB concentration, culture medium and culture temperature, which can be determined by one skilled in the art.

Typically, HAB are cultivated in the presence of the sample for at least 5 hours, and up to 24 hours. In some embodiments, HAB are cultivated in the presence of the sample between 8 and 16 hours.

The signal remains stable even after prolonged storage at room temperature, and thus samples can be read over a long time period.

HAB are cultivated in the presence of said sample, *i.e.,* the sample is deposited in or on the culture medium and HAB can access said sample, so as to use the protoporphyrin to allow or stimulate their growth.

### Step b) of the method

By "bacterial growth", it is intended in the present application the division of a given bacterial cell into two daughter cells. Accordingly bacterial growth therefore induces an increase of the bacterial biomass.

The "extent" of said bacterial growth corresponds advantageously to the increase in said bacterial biomass.

The extent of bacterial growth is estimated, and notably "visualized" or "measured", to obtain the said "signal".

In particular, by "sample signal", it is intended the extent of HAB growth, which is stimulated in the presence of the sample, and which is estimated (visualized and/or measured) at step b).

By "visualized" or "measured" extent of growth, it is intended advantageously as a direct or indirect eye visualization, and/or direct or indirect machine detection.

The said extent of HAB growth can be visualized (in particular for qualitative detection) and/or measured (in particular for semi-quantitative detection) according to classical methods of the art. For example, cell density in liquid medium is determined by a simple inexpensive spectrophotometer. In solid medium as used in the examples, HAB growth is seen as a halo around the wells within which the samples containing protoporphyrin are deposited. Gels can be scanned and signal density and diameters would correspond to the area of stimulated growth. In an alternative embodiment, cells can be genetically modified to express a fluorescent marker and the extent of HAB growth would be reflected by the intensity and area of the fluorescent signal. The use of such genetically modified HAB facilitates signal detection directly by visual inspection or using typically a fluorimeter, or even a simple UV box with a camera.

The nature of the signal thus depends on the methods used to estimate growth stimulation. Accordingly, any method commonly used for bacteriological growth readings and usable with the here-reported device can be employed.

Most easily among the methods referred to above, the extent of bacterial growth can be visualized by the formation of a halo (namely a change in the contrast/density of the medium) surrounding the heme-containing sample (*i.e.:* around the position where the sample was deposited). Therefore, the extent of bacterial growth can be estimated (or measured) by the contrast value and/or the size or surface of said halo surrounding the heme-containing sample. Hence the signal can consist in:
- optical density of the halo, or
- measurement of the surface area of the halo.

The intensity or level of said extent of bacterial growth (e.g.: optical density of the halo or surface area of the halo) can be advantageously detected by a machine, such as an adapted optical reader, a spectrophotometer, or a fluorimeter, as used to evaluate bacterial growth.

### Step c) of the method

In some embodiments, identification of the presence of protoporphyrin in a sample is achieved by the detection of a sample signal as mentioned as step b).

Preferentially however, qualitative or semi-quantitative detection of protoporphyrin in a sample is achieved by comparison of the sample signal obtained at step b) with at least one reference signal obtained from at least one reference sample. The said at least one reference signal can be predetermined, or determined in parallel to the sample signal.

A reference signal is obtained when HAB have access to a reference sample that is deposited in, or on, the culture medium, such that the growth of said HAB would be stimulated if the said reference sample contains protoporphyrin.

Reference signal(s) can be negative and/or positive reference signal(s). A negative reference sample gives a negative reference signal. A positive reference sample gives a positive reference signal.

By "standardized implementing conditions" it is herein intended the same type of HAB, and potentially the same cultivating time (namely the same "determined lapse of time" as used in step a) of the method) and/or the same protoporphyrin-lacking culture medium. The said references signals are therefore generally determined or predetermined, or theoretically determined, in the same standardized implementing conditions than the sample signal.

By "qualitative protoporphyrin detection", it is intended the determination of the presence, or the absence, of protoporphyrin, in the sample that is tested.

Typically, one negative reference sample and/or one or more positive reference sample(s) can be used, and the signals they generate are compared with the sample signal generated by the sample that is analyzed.

By "negative reference signal", it is intended the extent of growth obtained by culturing HAB in the protoporphyrin-lacking medium, but in the absence of the test sample (typically using a negative reference sample devoid of protoporphyrin). In practice, the negative reference signal is generally the signal that is generated by the overall background, as schematized in Figure 1A. It is noted that if a HAB uses heme but not PPIX, heme-specific detection is achieved, and the negative reference sample should be devoid of heme. On the contrary, if the HAB strain uses both heme and PPIX, the negative reference sample should be devoid of both heme and PPIX.

By "positive reference signal", it is intended the extent of HAB growth obtained by culturing HAB in the protoporphyrin-lacking culture medium, for a determined lapse of time, in the presence of a positive reference sample containing protoporphyrin. A positive reference sample can contain purified heme and/or PPIX and/or hemoglobin, and/or blood, as shown in Figure 2A. Alternatively, a positive reference sample may also be obtained by addition of purified heme, and/or hemoglobin, and/or PPIX, and/or blood to a sample (e.g., urine, feces, or gastric fluids) of a healthy patient, or from the tested patient, as shown in Figure 3A and 3B upper panels, respectively.

The comparison of the sample signal with the reference signal(s) at step c) consists advantageously in determining if the sample signal is different or identical to the said reference signal(s).

At least one negative reference signal produced by a negative reference sample can be compared at step c) with the sample signal, and can therefore be considered as a "negative threshold reference signal" (the negative reference sample being therefore considered in this case as a "negative threshold reference sample"). Said "negative threshold reference signal" corresponds to the signal intensity, above which, a positive result, namely the presence of protoporphyrin in the sample, is identified. If the HAB strain is activated solely by heme, the negative threshold reference sample should be devoid of heme. If the HAB strain is activated by heme and PPIX, the negative threshold reference sample should be devoid of both heme and PPIX.

In practice at step c) of the method, the presence of protoporphyrin is generally identified in the sample, if the sample signal is stronger than the negative threshold reference signal.

When the reference signal to be compared at step c) with the sample signal is a positive reference signal, the comparison therefore consists in determining if the sample signal is lower or higher than the said at least one positive reference signal.

Hence, at step a), in addition to the sample being tested, one would advantageously include at least one positive reference sample and/or one negative threshold reference sample.

The comparison at step c) of the at least one reference signal with the sample signal will therefore distinguish:
- a "positive" sample whereof the sample signal is higher than the negative threshold reference signal, corresponding to a sample comprising protoporphyrin, or having a level of protoporphyrin above that of the protoporphyrin threshold level, and
- a "negative" sample whereof the sample signal is the same as or lower than that of the negative threshold reference signal, and corresponding to a sample which does not comprise protoporphyrin, or has a protoporphyrin level that is identical or lower than said protoporphyrin threshold.

By "identify the presence of protoporphyrin", it is also intended that the invention is suitable for semi-quantitative protoporphyrin detection. Indeed, the performances of the method, or of the device according to the invention, also allow embodiments, wherein a semi-quantitative protoporphyrin measurement in a sample is achieved.

In these embodiments, the positive reference signal according to the invention can therefore be a benchmark reference signal. Typically such benchmark reference signal is established as mentioned above for the positive-reference signal, but by using a benchmark reference sample having a pre-determined concentration of protoporphyrin (heme and/or hemoglobin, and/or PPIX and preferentially heme alone).

In these embodiments the use of at least one benchmark reference sample allows one to attribute, by comparison of the at least one benchmark reference signal with the sample signal, a quantitative value of protoporphyrin to the analyzed sample.

Several benchmark reference samples, having each a different pre-determined protoporphyrin concentration (and generating each a different benchmark reference signal) can therefore be used. The amount of protoporphyrin in the sample is obtained by comparing the sample signal issued at step b) with said benchmark reference signals.

Advantageously, one benchmark reference sample may contain an amount of protoporphyrin considered as corresponding to the presence of a pathological amount of protoporphyrin. Said benchmark reference sample is therefore a positive threshold reference sample that generates a positive threshold reference signal. In this embodiment, if the sample signal is the same or stronger than the positive threshold reference signal, it is considered that the analyzed sample contains a pathological amount of protoporphyrin. At the contrary, if the sample signal is lower than the positive threshold reference signal, it is considered that the analyzed sample contains a non-pathological, or normal, amount of protoporphyrin (corresponding to "background" level protoporphyrin in sample)

Advantageously also, a benchmark reference sample may contain an amount of protoporphyrin correlated with a risk of disease, as known from the state of the art. In this embodiment, if the sample signal is the same or stronger than the positive threshold reference signal, it is considered that the analyzed sample contains an amount of protoporphyrin that is correlated with a risk of disease. At the contrary, if the sample signal is lower than the positive threshold reference signal, it is considered that the analyzed sample contains an amount of protoporphyrin that is not a risk of a disease.

For example, the cut-off value for a positive heme test using Hemoccult II® reportedly corresponds to about 300 µg hemoglobin, *i.e.,* about 12 µg heme per gram feces. In comparison, the test of the present invention detects less than 0.2 µg per gram feces. Thus, appropriate heme standards will give robust results using the present invention,

Preferentially, the sample signal is first compared with a negative threshold reference signal for qualitative result and further compared with at least one benchmark reference signal, in order to obtain a semi-quantitative determination of the amount of protoporphyrin in the sample.

The method according to the invention is therefore interesting in that it is suited for the qualitative detection of the presence of protoporphyrin, or of the specific presence of heme in a sample, namely the identification of a positive or a negative result, as well for the semi-quantitative determination of the amount of protoporphyrin, or specifically of heme.

The method of the invention can comprise a further step of identifying the presence of blood in said sample, in regard to the step c) of identification of protoporphyrin in the sample.

In practice, the said presence of protoporphyrin and notably of heme in a sample is correlated with the presence of blood in said sample.

### Sample according to the invention:

By « sample », it is intended according to the invention, any solid, semi-solid, or liquid specimens wherein protoporphyrin potentially present is to be detected.

For example the method allows detection of protoporphyrin and notably heme in any specimens. These include biological or bodily samples or environmental specimens such as food products or soil. Biological or bodily samples are for example feces (also named fecal material), blood, urine, saliva, or swab specimens of the cervix, urethra, nostril, or throat, bronchial lavage, pleural fluid, pulmonary fluid, synovial fluid, peritoneal fluid, gastro-intestinal fluids, abdominal fluids, or milk from a lactating mammal.

In preferred embodiments of the method, the sample is selected from feces, urine, gastro-intestinal fluids, abdominal fluids or milk.

Generally, samples to be tested according to the invention are complex samples comprising more than one compound. Contrary to biochemical or immunological tests, wherein complex samples may cause signal quenching, or where the detection threshold is high, in the method of the invention it has been demonstrated, as detailed in the experimental part, that the sample signal is not quenched by fecal material. Additionally, the signal does not fade with time (as is the case for Hemoccult II® test, for example).

In one preferred embodiment of the invention, the sample is heat-inactivated prior to step a) of cultivating HAB.

By "heat-inactivated", it is intended according to the invention any process that eliminates (removes) or kills microorganisms, without altering protoporphyrin present in the sample.

Heat-inactivation is achieved by heating the sample above 90 °C for 5 to 15 minutes, notably for 10 minutes.

Heat inactivation of the test sample increases its stability so that it can be preserved before testing, and may also limit any risks of contamination to the manipulator.

One interest of the method of the invention compared to immunologically-based tests is that it is insensitive to hemoglobin degradation.

### Culture medium

The culture medium used to grow the HAB protoporphyrin indicator strain can be any suitable medium for growing the HAB strains of interest, provided that it does not include significant levels of protoporphyrin. For heme-specific HAB as described above, the culture medium must not contain heme. When using HAB strains that are able to catalyze iron insertion in PPIX, the culture medium should be devoid of both heme and PPIX.

Non-limiting examples of suitable growth media include M17, MRS, BHI, or TH, which are common media for bacterial growth.

More particularly, the medium is a solid or a semi-solid medium. Preferentially, the culture medium is in the form of a gel. In a particular embodiment, the gel comprises between 0.2 % and 1 % agar, preferably at around 0.6 %.

The HAB are advantageously re-suspended in a homogeneous manner, in said culture medium. In the case of the gel setup, HAB are typically added just prior to pouring and solidification at a temperature between 30 and 50°C, preferably at 42°C, depending on temperature tolerance of the HAB strain.

The said HAB are incorporated at a concentration that is advantageously comprised between 10⁵ and 10⁶ colony-forming unit (cfu) / ml of culture medium.

### Devices and kits according to the invention:

The present invention also relates to kits and devices suitable for implementing the method as described previously.

A device according to the invention allows detection of the presence of protoporphyrin and, by direct correlation, allows detection of blood in a sample.

The said device contains HAB re-suspended in a protoporphyrin-lacking semi-solid or solid medium and the one or more samples are deposited in wells (Figure 1 A) or directly on the surface (Figure 1 B) of said medium.

In one embodiment (Figure 1A), the device comprises at least one well, named "sample well", intended to receive the analyzed sample, such that HAB, re-suspended in the medium as mentioned above, can access any protoporphyrin present in said sample to stimulate growth. Assessment of single or multiple samples on a single device having multiple wells (one well per sample) can be envisioned.

Preferentially, the device comprises also at least another well, named "reference well" used for the reference sample. In particular, the device may comprise at least one well intended to receive a benchmark reference sample having a predetermined protoporphyrin (preferentially heme or hemoglobin) concentration. The reference sample may be pure protoporphyrin, or protoporphyrin re-suspended in a sample devoid of protoporphyrin, for example, a sample of a healthy patient, to simulate the test sample environment.

Preferentially, said device comprises 1 to 5 reference wells, which are intended to receive benchmark reference samples having a series of predetermined protoporphyrin concentrations as above-mentioned, and as illustrated in Figure 2A.

Preferentially, the protoporphyrin-lacking medium is in the form of a gel, wherein the well(s) are directly formed. Preferentially, the device according to the invention is a gel-like apparatus wherein at least one well is formed for receiving the sample. In this embodiment, the gel comprises the protoporphyrin-lacking medium.

In another embodiment (Figure 1 B), the protoporphyrin-lacking medium is not pre-mixed with HAB, but may be deposited as a solid medium in a Petri dish. The HAB would then be either plated directly, or re-suspended in a gel overlay (referred to as "soft agar suspension" by those in the art), comprising the same protoporphyrin-lacking medium in approximately 0.5 to 0.8 % agar and deposited evenly on the solid medium.

In this case, depositing protoporphyrin or a sample containing protoporphyrin in a specific position on the plate will induce bacterial halo formation around the sample (schematized in Figure 1 B).

Preferentially, the device according to the invention allows direct visualization of bacterial growth around the test wells. Typically, when HAB are mixed in a gel medium, or plated directly above the medium and incubated under the appropriate growth conditions for the HAB being used, bacterial growth is visualized as a dense halo surrounding the protoporphyrin-containing sample.

A kit according to the invention comprises at least:
- the said device for implementing the method as described above
- a strain of HAB to be mixed with the protoporphyrin-lacking medium.
- at least one negative reference sample and/or at least one positive reference sample, and notably a benchmark reference containing a predetermined protoporphyrin concentration.

Preferentially, the kit comprises between two and five positive reference samples, each of which contains a different predetermined protoporphyrin concentration.

In one embodiment HAB are premixed with the protoporphyrin-lacking medium.

In practice, the implementation of such a kit comprises the following steps:
- if necessary, premixing HAB with the protoporphyrin-lacking medium, which is then deposited or spread in a gel-like apparatus for growth, with a space for sample wells (gel set-up illustrated in Figure 1A, plate set-up an alternative example in Figure 1 B).
- depositing the sample(s), and the reference sample(s) if necessary, and
- implementing the steps of the method according to the invention, to detect protoporphyrin in the said sample(s) in a qualitative and/or semi-quantitative manner.

### Use according to the invention:

The present invention is a highly sensitive and specific biological assay for determining the presence of protoporphyrin, and in particular the presence of heme and PPIX or the specific presence of heme, in a sample.

Said invention is particularly interesting as a diagnostic device in the pharmaceutical field. The said invention may also be used diagnostically to determine the presence of blood in a human or animal sample. The method of the invention can thereby be applied for preliminary screenings for potential inflammation or diseases such as colorectal cancer in the case of feces screening, hematuria in the case of urine screening or mastitis in the case of milk sample screening.

Furthermore, allowing detection of both heme and PPIX can further sensitize the test for cancer detection (Silva F R O et al., 2013, Journal of Fluorescence, 23(1):131-135). Porphyriarelated diseases or metal poisoning could also be detected (see Ng JC1 & Qi L, Moore MR Cell, 2002, Mol Biol ;48(1):111-23). Indeed in some embodiments, kits or devices according to the invention are used to determine protoporphyrin levels and notably heme levels in feces samples as a preliminary screening for potential inflammation or disease.

In some embodiments, the method of the invention or a kit according to the invention can be used in order to detect protoporphyrin and notably heme, and in particular the presence of blood, in a sample of milk from a lactating mammal. Said mammal is notably a farm animal and in particular a farm animal used for milk production.

The presence of protoporphyrin and notably heme, and in particular the presence of blood, in a sample of milk from a lactating mammal can be correlated to the presence of mastitis.

In other embodiments, the method of the invention or a kit according to the invention can be used in order to detect the presence of heme, and in particular the presence of blood, in a food product. The presence of heme or blood may be correlated to the presence of meat-derived ingredients (or substances from animal origin) in food products. The method of the invention can therefore be used, for the detection of meat-derived ingredients and notably for the detection of substances from animal origin, in a food product.

The method according to the invention can be easily adapted to various implementing conditions. In particular, the method of the invention allows protoporphyrin detection in a sample with simple equipment. By contrast, the method can be automated if suitable equipment is available in order to improve the reliability and the precision of the method and potentially increase the rapidity of reading results.

To summarize, the method of the invention combines both sensitivity and specificity, as well as signal stability. The method of the invention and the kit according to the invention are therefore particularly suited for early detection of intestinal inflammation and diseases and in particular of colorectal cancers, which is routinely based on a primary screen for heme or hemoglobin detection in feces samples. The method of the invention can be simply operated, using automated reading notably at steps b) and or c), or by visual detection, depending on the available equipment, and does not require expensive products. All these characteristics taken together make it particularly suited for use in primary large scale screening of colorectal diseases, such as adenoma, polyps and colorectal cancers.

Traces of heme might also reveal inflammation such as mastitis, a frequent and costly problem in farm animals such as cows and goats. Detection by the "HemeScreen" test could lead to early treatment to prevent spread of infection.

The present invention is further described in the following experiments that provide non limitative examples of implementing the invention.

### EXPERIMENTAL RESULTS

The test device of the invention is also named "HemeScreen" in the experimental part.

### Materials and methods:

*a) Gel-type approach:* Prototype device using *Bacteroides* growth stimulation as heme indicator: The device in this embodiment is schematized in Figure 1A. Briefly, a vertical gel is set up with a 0.8 cm spacer. Metal prongs (typically 4-5 cm deep) on a comb are used as wells.

*Bacteroides* cells (around 10⁵ to 10⁶ cfu/ml) are added at around 42°C just prior to pouring. Once solidified, wells are loaded (at positions of black arrows), and gels are overlaid with 5 ml of a 1.2% agar plug.

*Bacteroides* growth stimulation by test samples is visualized as dense growth forming a cream-colored halo of growth around wells after 12 to 16 hour (typically overnight) incubation at 37°C. Sample-generated signals can be compared to the negative reference signal corresponding to background, as schematized in Figure 1A.

A ∼0.5 to 1 cm no-growth-zone from the top of the gel is due to aerobic inhibition of *Bacteroides* growth. After incubation, the gel can be photographed for further analysis. The results are stable, and can be visualized over a period of several days.

b) *Petri plate approach:* Prototype set-up using *Lactococcus lactis* growth stimulation as heme indicator. The principle of the test is schematized in Figure 1B. Petri plates containing protoporphyrin-lacking solid medium are overlaid with about 10⁵ to 10⁷ cfu/ml of the HAB *(Lactococcus lactis)* re-suspended in soft agar (i.e., medium containing 0.75 % agar in typically 5 ml volume per standard Petri plate) and then poured over plates. After the soft agar solidifies, heme positive reference samples (*i.e.,* benchmark reference samples) and feces samples (between about 30 and 100 mg) are placed directly on plates, which are then incubated, usually overnight, at the optimal HAB condition and growth temperature (as per current knowledge of the HAB, or determined empirically). Stimulatory effects of heme in samples are then observed visually and are photographed or scanned.

Samples of intestinal contents were obtained from healthy conventional and germfree mice routinely raised in the animal facilities. Meconium samples used in the present study were collected from human newborns, and fecal samples were obtained from human adult volunteers. All samples were immediately used or stored at -80°C prior to use.

Fecal samples were routinely heated to 90°C for 10 minutes to avoid outgrowth of endogenous bacteria. Samples were routinely re-suspended 10 % weight/volume suspension in NaCl (0.9 %) and then heated. Volumes used as samples were 50 to 100 µl, and correspond to about 5 mg feces per sample, as tested in setup described in Figure 1A. Samples tested in setup described in Figure 1B were used directly, such that 50-100 mg feces were deposited on plates for testing.

*c) Additives used in growth assays.* Heme, PPIX, biliverdin, and bilirubin were prepared as 20 mM stock solutions as described (Fernandez et al., 2010 Plos Pathogens, 6(4):e1000860). Hemoglobin was prepared as a 10 mg/ml H₂O stock solution. Products were purchased from Sigma. Horse blood dilutions were prepared in 0.9 % NaCl.

*d) Strains and growth conditions: B. thetaiotaomicron* reference strain VPI-5482 (provided by Sylvie Rabot, INRA, France), and *Bacteroides fragilis* strain ADB77 provided by Michael Malamy (Tufts University, USA) were used as HAB. These *Bacteroides* cultures were routinely started from 50 µl frozen aliquots prepared as follows: An overnight culture was prepared in M17 plus 0.5 % glucose (M17-glu) containing heme (10 µM) and cysteine (4 mM) and grown anaerobically at 37°C; the culture was washed and re-suspended in fresh M17-glu medium containing 15 % glycerol and then aliquoted anaerobically and frozen. For all experiments, *Bacteroides* cultures were prepared as follows: 10 µl of a frozen aliquot was thawed and re-suspended in 10 ml M17-glu (without heme) and grown overnight at 37°C in anaerobic conditions, using either an anaerobic chamber or jar. The strain is routinely started from a frozen culture and grown aerobically overnight in static conditions in M17-glu at 30°C. *Lactococcus lactis* was grown as routinely described (Duwat et al., J Bacteriol., 2001, 183:4509-16).

### Results:

The device is set up to use an easily cultivated bacterium whose growth requires exogenous heme. *Bacteroides (thetaiotaomicron or fragilis*) have been used due to their significant growth stimulation by low amounts of heme. Although these bacteria are considered as anaerobes, they tolerate low amounts of oxygen, which makes handling straightforward, and they grow rapidly (*i.e.,* to saturation upon overnight growth). Oxygen-tolerant *B fragilis* variants were reported, due to acquisition of mutations in a conserved flavoprotein (see Meehan et al., 2012, PNAS, 109:12153-58). *B. thetaiotaomicron* and *B fragilis* oxygen-tolerant variants may be used as indicator strains in the present protoporphyrin detection tests.

*a) The method is highly specific for heme*/*PPIX* According to Figure 2A, different concentrations of: purified heme (upper panel), ranging from 0.5 to 256 ng of heme; hemoglobin (middle panel), ranging from 1 to 500 µg; or horse blood (lower panel) ranging from 12 to 6000 nl, were loaded in wells, and growth stimulation was evaluated. Heme-mediated growth stimulation was observed with as little as 1 ng heme as detected by dense growth surrounding sample wells. Hemoglobin-mediated HAB growth stimulation was observed with as little as 2 µg of hemoglobin and blood-mediated mediated HAB growth stimulation was observed with as little as 12 nl of blood.

According to Figure 2B, PPIX, the iron-free heme precursor also stimulated *B. thetaiotaomicron* (Bt) growth. The detection of both heme and PPIX suggests that *Bacteroides* encodes enzymatic activity allowing it to insert available iron into PPIX (see Sperry JF et al., 1977, Appl. Environ. Microbiol., 34:386-90). Studies in *E. coli* revealed a novel enzyme that is needed to remove iron from heme (Letoffe et al., 2009, PNAS, 106:11719-24). A homolog having potentially reversible activity may be present in *Bacteroides.* Its inactivation could be useful to restrict detection to heme.

In contrast, bilirubin or biliverdin, which are heme breakdown products that may also be present in feces, did not impact on Bt growth, even at high concentrations (Figure 2B), attesting to the specificity of the test for protoporphyrins.

*b) Heme detection in feces.* In Figure 2C, feces samples were heated to 90°C for 10 minutes. It has been shown that heat treatment does not alter Bt growth stimulation but rather stabilizes the test and avoids bacterial contamination and gas bubbles due to fermentation, therefore improving quality of results.

Feces from healthy germfree or conventional mice (GF or CONV respectively), as well as samples from human meconium or adult (M or AD respectively), were deposited in wells (5 mg feces re-suspended to 50µl 0.9 % NaCl). At left, heme (120 ng, which is within the range of heme present in healthy individuals) was used as positive reference. The results showed that all feces samples from healthy individuals stimulated Bt growth. Specificity of heme stimulation was verified separately and allows one to conclude that heme or PPIX is responsible for the observed stimulation.

*c) Semi-quantitative detection of heme in complex biological materials such* as *urine and feces. Signals are not quenched when measured in biological samples, and are more sensitive than conventional heme detection tests.*

50 µl urine (Figure 3) or 5 mg feces suspended to 50 µL in 0.9 % saline (Figure 4) from healthy individuals was spiked with different heme concentrations such that the amounts of heme per above-stated aliquots corresponded to between 1 ng and 256 ng in Figure 3, and to between 4 ng and 1024 ng in Figure 4.

Figure 3: The spiked urine samples were loaded on the "HemeScreen" gel setup of the invention (3A UPPER), and the same sample was tested using Multistix® 8SG, which is routinely used to detect hematuria (3A LOWER).

Figure 4: The spiked fecal samples were loaded on the "HemeScreen" gel setup of the invention (4 UPPER), and the same sample was tested using Hemoccult II® (4 LOWER).

Note that feces for the "0" point in Figure 4 is positive, consistent with the known and observed presence of trace heme in healthy individuals as also seen in Figure 2C. Growth stimulation is increased particularly when 128 ng heme are added to the sample, which indicates that this amount of heme can be distinguished from the amount present in the sample. Thus levels exceeding 128 ng of heme will be detectably higher than the amount of heme present in this normal feces sample. The results show that heme amounts can be quantified at very low levels, regardless of the complexity of the biological material, and even in healthy individuals.

It is well known that complex biological samples, especially feces, cause signal quenching when heme is measured biochemically.
Biochemically-based kits are available to quantify heme, either pure or in feces samples. We showed that purified heme was measurable, but as reported, signals diminish when feces were added to heme samples. A similar problem was reported for an immunological test; signals were 90-fold lower in samples containing feces, when compared to purified hemoglobin (Vaananen O & Tenhunen R, 1988, Clin Chem., 34:1763-66).

Importantly, and favoring the "HemeScreen" test of the invention, heme detection levels are not diminished by the biological material in which heme is present; indeed, the presence of complex samples such as urine or feces material does not quench the heme signal since increases in heme amounts in the samples are readily detected even at low levels.

Using the plate set-up (schematized in Figure 1 B), another HAB was tested to detect the presence of heme (Figure 5). Upper panels show heme (120 ng) and different feces samples (between about 30 and 100 mg) deposited on an overlay of *L. lactis* strain MG1363. The halo surrounding the samples indicates growth stimulation of *L. lactis.* Lower panels show the same samples deposited around the MG1363 *cydA* strain, which is unable to use heme (Duwat et al., 2001, J Bacteriol., 183:4509-16). There is no stimulation by heme, nor by the test samples, showing specificity of the test.

Taken together, the above mentioned results show that heme can be readily evaluated using a bacterial-growth-based assay that is simple to set up at a low cost.

## Claims

1. A method for detecting the presence of protoporphyrin in a sample, said method comprising steps of:
a) cultivating heme auxotroph bacteria (HAB) in a protoporphyrin-lacking culture medium, in the presence of said sample, for a determined time period,
b) estimating the extent of HAB growth issued at step a), to obtain a sample signal, and
c) identifying the presence of protoporphyrin in said sample, by comparing the sample signal obtained at step b) with at least one reference signal.

2. A method according to claim 1, wherein the protoporphyrin is heme and wherein at step a), HAB are heme-specific HAB which are cultured in a heme-lacking medium.

3. A method according to claim 2, wherein heme-specific HAB are chosen among native or recombinant strains that are deficient in enzymes that catalyze iron insertion into protoporphyrin IX.

4. A method according to anyone of claims 1 to 3, wherein the reference signal consists in one negative reference signal and/or at least one positive reference signal.

5. A method according to anyone of claims 1 to 4, further comprising a step of estimating the amount of protoporphyrin in the sample by comparing the sample signal obtained at step b) with at least one benchmark reference signal.

6. A method according to anyone of claims 1 to 5, wherein HAB are chosen among the genera *Bacteroides, Haemophilus, Lactobacillus, Lactococcus, Enterococcus,* heme auxotrophic mutants of *Escherichia,* heme auxotrophic mutants of *Bacillus,* or their recombinant HAB derivatives.

7. A method according to anyone of claims 1 to 6, wherein the sample is a complex sample, preferentially a food product such as milk, fecal material, a gastro-intestinal fluid, or urine.

8. A method according to anyone of claims 1 to 7, further comprising a step of heat-inactivating the sample, prior to step a).

9. A method for detecting the presence of blood in a sample, said method comprising:
- steps of the method according to any one of the claims 1 to 8, and
- a further step d) of identifying the presence of blood in said sample, in regard of step 1c) of identification of protoporphyrin in the sample.

10. A device for implementing a method according to any one of claims 1 to 9.

11. A device according to claim 10 containing HAB suspended in a protoporphyrin-lacking culture medium and at least one well intended to receive a sample, wherein said HAB can access to said sample.

12. A device according to claim 11 wherein the protoporphyrin-lacking culture medium is in the form of a gel.

13. A device according to claims 10 to 12, further containing at least one other well, which is intended to receive a reference sample.

14. A kit comprising:
- a device according to any one of claims 10 to 13,
- at least one benchmark reference sample having a predetermined protoporphyrin concentration, and
- a strain of HAB to be mixed with the protoporphyrin-lacking medium.

15. Use of a device according to claims 10 to 13 or a kit according to claim 14 for preliminary screening for potential inflammation or disease.

16. Use of a device according to claims 10 to 13 or a kit according to claim 14 for detection of mastitis in farm animals, especially those used for milk production.

17. Use of a device according to claims 10 to 13 or a kit according to claim 14 for detecting the presence of meat-derived ingredients in food products.
